Europäisches Patentamt

European Patent Office

Office européen des brevets

(11) Publication number: **0 485 859 A2**

# EUROPEAN PATENT APPLICATION

(21) Application number: **91118811.8**

(51) Int. Cl.5: **C02F 3/12, B01F 3/12**

(22) Date of filing: **05.11.91**

(30) Priority: **13.11.90 IT 2203490**

(43) Date of publication of application:
**20.05.92 Bulletin 92/21**

(84) Designated Contracting States:
**AT BE CH DE DK ES FR GB GR IT LI LU NL SE**

(71) Applicant: **ECOLMARE S.p.A.**
**Via delle Rose, 50/a**
**I-80063 Piano Di Sorrento ( Prov. of**
**Naples)(IT)**

(72) Inventor: **Kimchie, Shlomo**
**Harrishonim 72a St.**
**Kiyat Haim, Haifa IL-26302(IL)**
Inventor: **Ben-Avrahim, Akiva**
**115, Yosephtal Ave.**
**Bat Yam IL-69548(IL)**
Inventor: **Shelef, Gedalish**
**Ayalon 9 St.**
**Haifa IL-34336(IL)**

(74) Representative: **Gervasi, Gemma et al**
**NOTARBARTOLO & GERVASI Srl Viale**
**Bianca Maria 33**
**I-20122 Milan(IT)**

(54) **Rotating device with centrifugal sludge lifting for bioreactors.**

(57) A rotating device is described for the centrifugal sludge lifting in bioreactors, particularly suitable for carrying out biological processes under mixing conditions presenting extremely low shearing forces.

The device comprises a hollow vertical rotating shaft (3) and at least one rosette (4) of horizontal tubular arms solidly connected with said vertical shaft (3).

FIG 1

Rank Xerox (UK) Business Services

(-/2.18/2.0)

## Background of the invention and prior art.

The present invention relates to a rotating device whereby sludges in bioreactors may be centrifugally lifted, and which is particularly useful in biological processes.

In bioreactors a mixing is normally required to accelerate the mass and heat transfer processes. The most common mixing types are: mechanical, pneumatic and hydraulic. Numerous variations are possible for each type.

In the many publications dealing with the effect of shear forces on the microbial activity, a wide range of sensitivities as well as great changes in them according to growth phases, growth cycles and other growth conditions are given.

The efficiency of many biotechnological processes depends on the behavior of bacterial flocs or of other types of bacterial aggregations, such as biomass granules or biological slimes.

In many such cases, relatively small shearing forces may damage the aggregate's structure and reduce the efficiency of the process.

Many mixing devices, particularly those intended for sludges, are therefore structured in such a way as to produce the maximum admixing of the liquid while exerting a minimum of shear stress on the biomass.

This is achieved by reducing the collision forces in the interface between moving parts of the mixing devices and the biomass particles (sludge flocs, single cells, etc.).

Another attitude for reducing shearing forces in reactors is to avoid high hydraulic flow velocities at critical points of the mixer, e.g. near liquid jet nozzles, gas nozzles, inside pumping tubes, etc.

In general, one of the basic problems in the mixing of biological systems is that it also creates in the reactor shearing forces which might cause damage to the microorganisms and slow down their activity.

## Detailed description of the invention

The present invention relates to a rotating device for centrifugally lifting sludges in bioreactors, consisting of a rotating vertical hollow shaft and of at least a rosette of horizontal tube arms solidly connected with said vertical shaft.

The rotation of said device creates a centrifugal force at the ends of the horizontal tube arms. Said force lifts the liquid (together with the sludges, cells, etc.) from the bottom of the shaft and discharges it through the tips of the horizontal tube arms.

With a tube arm system consisting of tubes of large diameter it is possible to obtain a high flow in the arms also at low rotation velocities.

According to a typical characteristic of the device according to the invention, the ratio between the section of the internal shaft cavity and the sum of the sections of the rosette tubes is such that the centrifugal effect obtained during rotation of the shaft imparts a linear lifting velocity to the sludge inside the same comprised between 0.1 and 1 m/sec.

In the preferred embodiments of the device according to the invention, said ratio between tube sections is comprised between 1:2 and 1:30 and the shaft rotates at a velocity of between 1 and 20 turns per minute.

With the device according to the invention it is thus possible to reach maximum pneumatic turbulence in the liquid with a minimum of damages by the shearing force created by moving parts or by excessive liquid flow velocities.

The device according to the invention can therefore find wide application in numerous biotechnology fields, particularly for processes employing submerged tissue cultures; in biotechnological processes for environmental defense; in processes based on sludges sensitive to shearing forces, such as contact anaerobic processes and other high yield anaerobic processes based on short hydraulic retention time and high solid retention time; in high-rate nitrification and denitrification processes in reactors with long solids (biomass) retention times.

In most cases where the device according to the invention is employed, it constitutes the unique mixer for a bioreactor containing sludges, cells and particles of materials which may sediment and on which are immobilized microorganisms, enzymes or biomasses of any other form which are sensitive to shearing forces.

According to a typical embodiment of the invention, the device is installed in multiple compartment bioreactors, in which it acts as a controlling means of the movement of sludges and liquids within a same compartment and between different compartments.

## Brief description of the drawings

Fig. 1 - Schematic representation of a reactor equipped with the rotating centrifugal sludge lifting device.

Fig. 2a - 2d - Schematic views of the horizontal tube arm system of the device represented in Fig. 1 Section A-A.

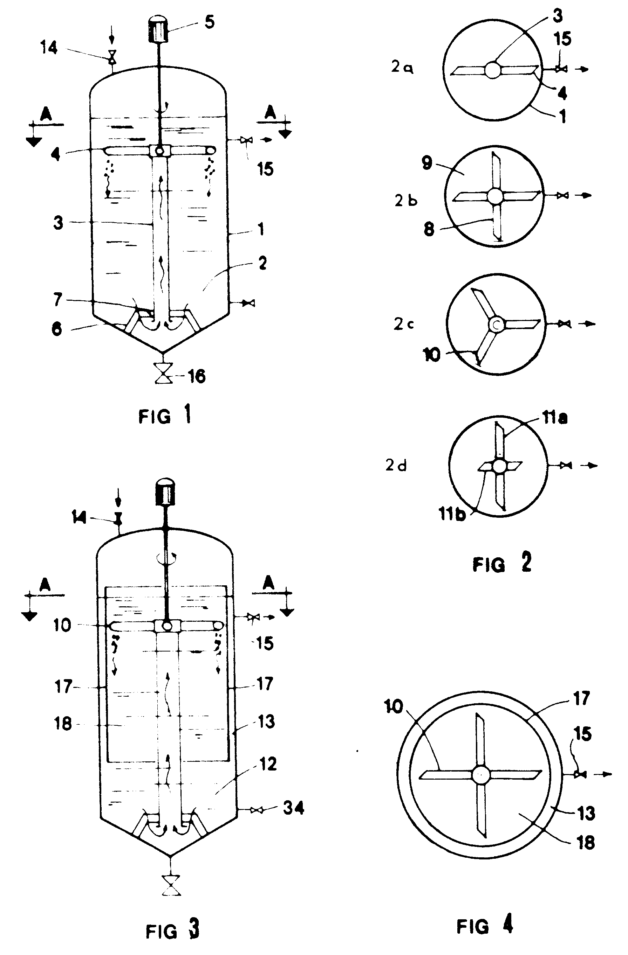

Fig. 2a - Two arm rosette.

Fig. 2b - Four equal arm rosette.

Fig. 2c - Three equal arm rosette.

Fig. 2d - Rosette with four arms (two pairs)

Fig. 3 - Schematic representation of a reactor with rotating centrifugal sludge lifting device and internal settler.

Fig. 4 - Schematic sectional view of the horizontal tube arm device and settler of the reactor shown in fig. 3, Section A-A.

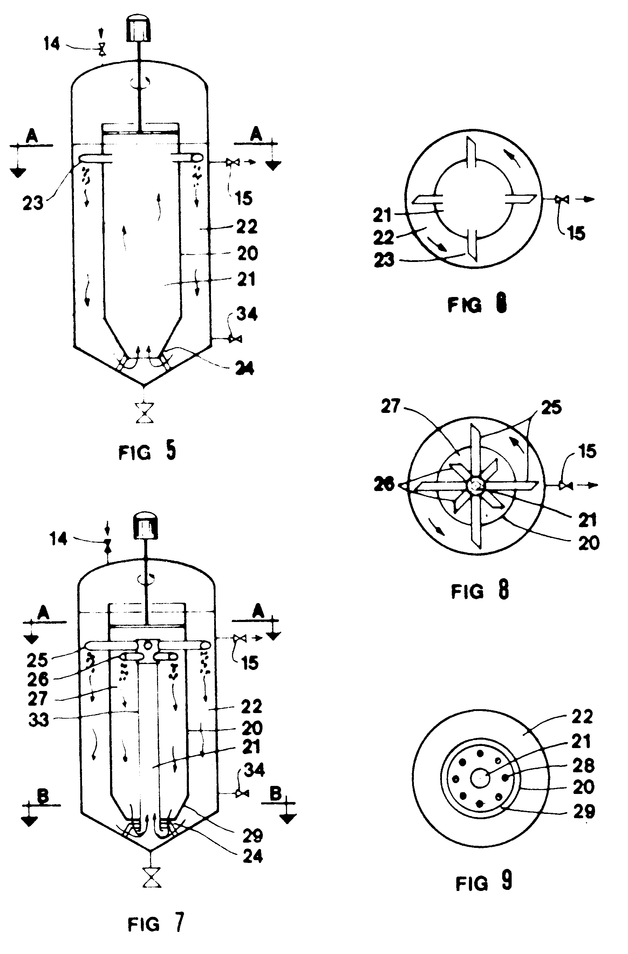

Fig. 5 - Schematic representation of a two compartment reactor with integrated rotating centrifugal sludge lifting device.

Fig. 6 - Schematic sectional view of the horizontal tube arm system and of the two compartments of the reactor shown in Fig. 5, Section A-A.

Fig. 7 - Schematic view of a reactor divided in two compartments with integrated rotating centrifugal sludge lifting device and optional sludge mixing in each compartment.

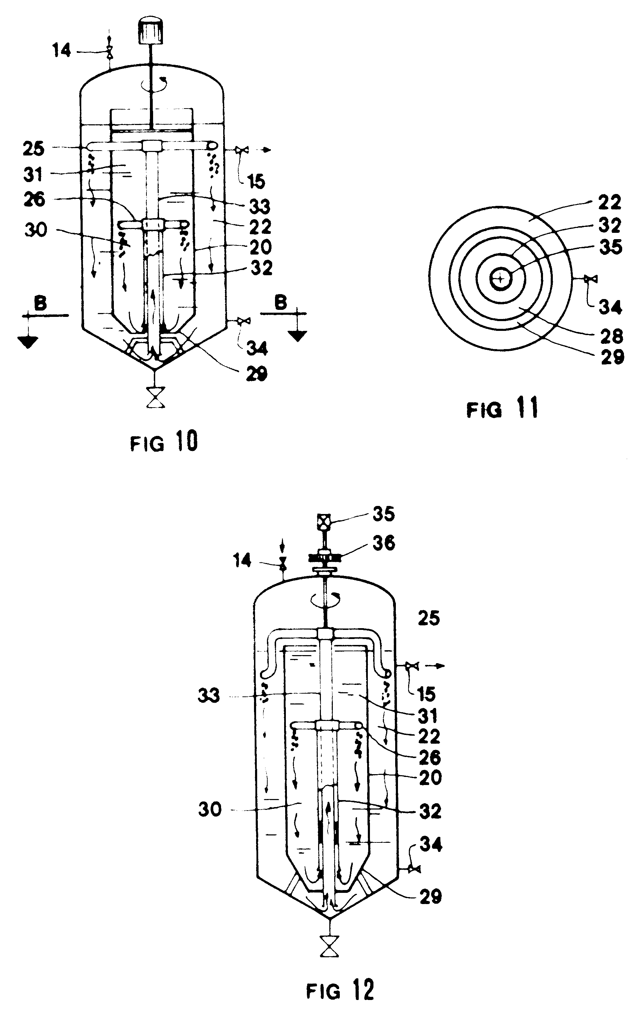

Fig. 8 and 9 - Schematic sectional views od a horizontal tube arm device, of the two compartments and of the bottom of the reactor internal compartment.

Fig. 10 - Schematic representation of a reactor divided into two compartments and rotating centrifugal sludge lifting device with independent sludge mixing in each compartment.

Fig. 11 - Schematic, sectional view of the internal compartment bottom in the reactor of Fig. 10, Section B-B.

Fig. 12 - Schematic representation of a reactor divided into two compartments with integrated rotating centrifugal sludge lifting device and independent sludge mixing in the two compartments.

With particular reference to the attached figures, we will now describe some preferred embodiments of the device according to the invention.

Fig. 1 shows the essential portion of the centrifugal sludge and cell lifting device installed inside the reactor 1,2 comprising a vertical cylindrical hollow shaft 3 and a rosette of tubular horizontal arms 4 mounted at the top of the shaft 3. The device is wheeled by a motor 5.

As a result of the centrifugal force that acts on the liquid at the tip of the arms, a pumping action is formed which pumps the sludge or the cells from the bottom of the vertical shaft 3 to the tips of the arms 4. The sludge that settles on the bottom of the reactor 1 is lifted to the top of the reactor and from there settles again toward the reactor bottom.

In the cases where a sludge retention time longer than the hydraulic retention time is desired, it is possible to combine said reactor with a sludge recovery device (settling tank, centrifuge, etc.) and have the sludge returned into the reactor. Another possibility is to operate the reactor on the basis of intermittent fill and draw cycles, in which the effluent draw phase from the reactor is followed by a resting phase for the sludge centrifugal lifting device, which allows for sludge settling in the reactor. Said cycles may be easily controlled by an automatic timing system of conventional type.

Fig. 2 shows several types of rosettes with different numbers of horizontal arms and different arrangements of them, in particular:

Fig. 2a shows an arrangement with two arms; Fig. 2b one with four;

Fig. 2c one with three and Fig. 2d one with four in a two pair arrangement 11a and 11b.

Through these arrangements a more homogeneous distribution of the sludge is obtained, which settles on the entire section of the reactor.

Fig. 3 shows a reactor presenting a device for the centrifugal sludge lifting in which the effluent leaving the reactor through the discharge valve 15 is the supernatant layer from an internal settler 17. The settler may be a concentric cylinder or part of a cylinder which protects the opening of the discharge valve 15 from the penetration of sludge particles lifted by the centrifugal sludge lifting device flowing through the arms of the horizontal tube system 10.

If the tubular arm rosette is not situated at the top of the reactor, but at a lower level, a vertical division of the reactor volume is obtained, the lower part rich in suspended particles or sludge, while the higher contains a relatively clear liquid.

Fig. 4 is a top view of the settler at the level of the overflow valve 15 (Section A-A in fig. 3) showing the complete separation between the mixed sludge cell 18 and the cell 13 of the effluent. In comparison to the configuration shown in Figs. 1, 6, 7, 10 and 12, the configuration including an internal settler, such as the one of fig. 3, has the advantage of continuous sludge separation through the intermittent fill and draw system without the need of further devices.

Fig. 5 shows a configuration of the centrifugal sludge lifting device according to the present invention in which the vertical hollow shaft 20 has a diameter such as to divide the reactor in which it is installed into two compartments of more or less equal volumes, a central one consisting of the shaft cavity and the peripheral one formed by the space between the shaft and the reactor walls, so that the reactor contents are lifted from the bottom through the internal shaft cavity and are discharged in the peripheral compartment through the tubular horizontal arms.

Both compartments contain a same sludge, which flows upward in the internal compartment 21 and then downward (by effect of gravity) in the peripheral compartment 22.

The advantage of this configuration is that it creates in the reactor liquid a plug flow like regime.

The direction of flow is from the inflow point 14 in the internal compartment 21 and then through the reactor's bottom in the direction of the discharge point 15 situated in the upper part of the peripheral compartment.

The centrifugal sludge lifting device maintains a more or less uniform sludge distribution throughout the reactor volume.

Fig. 6 shows a schematic top view of a possible configuration of the horizontal tubular arms shown in Fig. 5 (Section A-A).

Fig. 7 shows the possibility of dividing the reactor volume into two compartments without using the vertical shaft of the centrifugal sludge lifting device as the inner cell.

In this configuration, the volume of the inner cell 27 is separated from the volume of the peripheral cell 22 by an internal cylinder 20. The inner part of the hollow shaft 21 acts as the common transportation duct for the sludge, from which it is distributed into both the inner and peripheral compartments, 27, 21 respectively. The proportion in which the sludge will be distributed between 27 and 21 is pre-designed by the number of horizontal tube arms in the inner and peripheral rosettes, 26, 25 respectively and by their geometry (diameter and length). The inner cell 20, together with the centrifugal sludge lifting device, co-rotates using special bearing 24 near the bottom of the tank.

Fig. 8 discloses a possible configuration of the arms 25, 26 in the horizontal tube rosette. Fig. 9 shows the perforations in the bottom of the inner compartment whereby the sludges of the two compartments 22 and 27 mix together. Fig. 9 discloses an array of opening 28 in the bottom of the inner compartment which permits passage and mixing of the sludges between the two compartments of the tank 22, 27. The advantage of the configuration shown in figs. 7, 8 and 9 is that it permits an independent and controllable sludge-lift mixing in each of the reactor's compartments.

Fig. 10 and 11 disclose a configuration in which the inner 30 and peripheral 22 compartments of the reactor do not have free sludge passage at the reactor's bottom such as that in the configuration shown in Figs. 7, 8 and 9.

The centrifugal sludge lifting device shown in figs. 10 and 11 has a shaft with two co-axial cavities 32, 33 which do not communicate with each other and are connected to two different horizontal tubular arm rosettes which also are unconnected with each other. The more external coaxial cavity 32 of said shaft lifts the sludge from the bottom of the inner compartment 30 and lifts it to the height of rosette 26 located at the lowest level, the tips of the horizontal tubular arms pouring the lifted sludge into said inner compartment 30, while the interior co-axial cavity 33 of said shaft takes the sludge from the bottom of the peripheral compartment 22 and lifts it up to the rosette situated at a higher level, the tips of the horizontal tubular arms pouring the lifted sludge into said peripheral compartment 22.

In the example shown in Fig. 10, the internal tubular horizontal arm rosette 26 is situated approximately at half height in the reactor, but may be situated at any height, according to need.

The advantage of the configuration shown in Figs. 10 and 11 is that a process which is based on two different kinds of sludges may be held simultaneously in one reactor tank. Openings between the inner and peripheral compartments, which are penetrateable by the liquid but not by the sludge may form a directed hydraulic flow between the two compartments. Such a flow may permit in one reactor tank a sequence of reactions, carried out simultanously by two pre-selected sludges.

Fig. 12 discloses a configuration in which the reactor tank is separated into two compartments by an inner cylinder 20. In contrast to other "two-cells" configurations shown earlier in figs. 5 to 11 of the present invention, in which the cells' separating cylinder co-rotates with the centrifugal sludge lifting device's shaft, the cylinder 20 in Fig. 12 is fixed in place. The arms of the upper horizontal tubular arm rosette which serve the sludge lift of the peripheral cell are located mainly above the level of the cylinder 20 and only the arms' tips are bent down into the liquid.

Once the tubular horizontal arms 25 are full of liquid, they remain full until some lowering of the pressure or gas formation or penetration take place in that part of the centrifugal sludge lifting device or in the shaft 33.

To prime the centrifugal pump effect of the device for the centrifugal sludge lifting of the present invention, are to overcome casual pressure leakages, the device is equipped with a water filling device and a one-way valve 35 on top of the hollow shaft. The pulley 36 in this configuration will have to be located in a lower part of the shaft.

The main advantage of the configuration in fig. 12 is the saving it allows in energy and construction costs avoiding the rotation of large and heavy cylinders such as those required to separate inner from peripheral cells of large reactors.

A further advantage of the configuration shown in fig. 12 is that most of the horizontal tubular arm rosette 25 rotates outside of the liquid, with minimum friction and turbulence.

## Claims

1. Rotating device for the centrifugal lifting of sludges in bioreactors, consisting of a vertical, hollow rotating shaft (3) and at least one rosette (4) of tubular horizontal arms solidly connected to said vertical shaft (3).

2. Device according to claim 1, characterized in that the ratio between the sections of the internal cavity of said shaft (3) and the sum of the sections of the horizontal tubular arms (4) is such that the centrifugal effect obtained during the shaft (3) rotation imparts to the sludge inside the same a linear lifting velocity of between 0.1 and 1 m/sec.

3. Device according to claim 1, characterized in that the ratio between the section of the internal cavity of said shaft (3) and the sum of the sections of the horizontal tubular arms (4) is comprised between 1:2 and 1:30 and the shaft (3) has a rotation velocity of between 1 and 20 turns per minute.

4. Device according to claim 1, characterized in that it forms the only mixer in a bioreactor containing sludges, cells or material particles which may settle and on which microorganisms, enzymes or biomasses of any other type sensitive to shearing forces are immobilized.

5. Device according to claim 1, characterized in that said hollow, vertical shaft (20) has a diameter such as to divide the reactor volume in which it is installed into two compartments of more or less equal volumes, one of which, central, consisting of the shaft cavity (21), and the other, peripheral (22), consisting of the space comprised between the shaft and the reactor walls, so that the reactor contents are lifted from the bottom through the internal cavity (21) of said shaft and discharged through the horizontal tubular arms in the peripheral compartment.

6. Device according to claim 1, characterized in that said shaft is mounted in a reactor divided into two concentric compartments; that said shaft has two co-axial cavities not connected with each other and connected to two different rosettes (25, 26) of horizontal tubular arms which are equally unconnected with each other; that the more external co-axial cavity of said shaft takes the sludge from the bottom of the internal compartment (27) and lifts it to the height of the rosette (26) situated at the lowest level, the tips of the horizontal tubular arms pouring the lifted sludge into said internal compartment (27), while the more internal co-axial cavity (21) of said shaft takes the sludge from the bottom of the peripheral compartment (22) and lifts it up to the rosette (25) situated at a higher level, the tips of the tubular, horizontal arms of which pour the lifted sludge into said peripheral compartment (22).

7. Device according to claim 1, characterized in that said rosette (4) is not situated at the summity of the reactor, but at a lower level, thus forming a vertical division of the reactor volume into a lower part rich in suspended particles or sludge, and a upper part of relatively clear liquid.

8. Device according to claim 1, characterized in that said horizontal tubular arm rosette (4) is situated mainly above the level of the liquid contained in the reactor, and only the tips of said arms are bent and dip into the liquid.

9. Device according to claim 1, characterized in that said hollow shaft (3) has a water admission system and a one way valve (35) at the summit to prime the device itself at the start and in case of stoppage.

FIG 1

FIG 2

FIG 3

FIG 4

FIG 5

FIG 6

FIG 7

FIG 8

FIG 9

FIG 10

FIG 11

FIG 12